# FASCICULE DE BREVET EUROPEEN

(11) **EP 3 099 221 B1**
(45) Date de publication et mention de la délivrance du brevet: **08.11.2017**
(21) Numéro de dépôt: 15705656.5
(22) Date de dépôt: 23.01.2015
(51) Int. Cl.: A61B 5/00, G01N 21/11, G01N 21/35, G01N 21/55, G01N 33/49, G01N 21/03, G01N 21/43, G01N 21/49, G01N 21/47, G01N 21/552, G01N 21/359, B01L 3/00, G01N 21/3577, G01N 33/487, G02B 6/02

(54) **DISPOSITIF A ONDES ÉVANESCENTES, ET PROCÉDÉ DE MISE EN OEUVRE**
VORRICHTUNG ZUR ERZEUGUNG VON EVANESZENTEN WELLEN UND VERFAHREN ZUR IMPLEMENTATION DAVON
DEVICE GENERATING EVANESCENT WAVES, AND METHOD FOR THE IMPLEMENTATION THEREOF

(30) Priorité: 27.01.2014 FR 1450661
(43) Date de publication de la demande: 07.12.2016
(73) Titulaire: Diafir, 35000 Rennes (FR)
(72) Inventeur: TARIEL, Hugues, F-35000 Rennes (FR); CHARPENTIER, Frédéric, F-35000 Rennes (FR)
(74) Mandataire: Jolly, Christophe
(86) Numéro de dépôt international: PCT/FR2015/050168
(87) Numéro de publication internationale: WO 2015/110767

(56) Documents cités:
- US-A1- 2011 301 047
- MARIE-LAURE ANNE ET AL: "Fiber evanescent wave spectroscopy using the mid-infrared provides useful fingerprints for metabolic profiling in humans", JOURNAL OF BIOMEDICAL OPTICS, vol. 14, no. 5, 2009, pages 054033-1-054033-9, XP055135670, ISSN: 1083-3668, DOI: 10.1117/1.3253319
- SHUO CUI ET AL: "From Selenium- to Tellurium-Based Glass Optical Fibers for Infrared Spectroscopies", MOLECULES, vol. 18, no. 5, 10 mai 2013 (2013-05-10), pages 5373-5388, XP055135674, DOI: 10.3390/molecules18055373

## Description

La présente invention concerne un dispositif à absorption d'ondes évanescentes ainsi qu'un système de spectrométrie comprenant un tel dispositif et un procédé pour la mise en oeuvre du dispositif.

Depuis plus d'une décennie, la communauté scientifique et médicale s'intéresse de plus en plus au développement de méthodes non-invasives de diagnostique utilisant des fibres optiques.

En particulier, les capteurs à ondes évanescentes utilisés pour mettre en oeuvre une technique de spectrométrie infrarouge se sont particulièrement développés. Ce type de technique tire son intérêt de l'utilisation d'une fibre dans laquelle une onde évanescente parcourt la surface extérieure de la fibre lorsqu'une lumière infrarouge se propage dans la fibre.

Lorsqu'un échantillon biologique est mis en contact avec un guide d'onde, l'onde évanescente interagit avec l'échantillon biologique, ce qui conduit à une absorption de certaines longueurs d'ondes de la lumière infrarouge évanescente.

L'analyse par spectroscopie consiste ensuite à comparer le spectre obtenu avec et sans échantillon liquide au contact de la surface externe du guide d'onde et à en déduire les substances présentes dans l'échantillon.

Pour réaliser une analyse spectroscopique, on utilise habituellement la gamme d'infrarouge moyen s'étalant approximativement de 800 à 10000 cm⁻¹ ou encore lointain qui s'étale approximativement de 400 à 800 cm⁻¹.

En particulier, il est connu d'utiliser un capteur à ondes évanescentes se présentant sous la forme d'une fibre optique laquelle a habituellement un diamètre de quelques centaines voire quelques dizaines de micromètres, le diamètre n'étant pas forcément constant le long de la fibre.

Il est connu du document WO2011/121086 de courber au moins une partie d'une fibre optique de manière à réaliser des distorsions de propagation des ondes dans la fibre, dans le but d'augmenter localement au niveau de la partie courbée le taux d'ondes évanescentes et ainsi augmenter la sensibilité de détection des composés dans un échantillon biologique.

Toutefois, les capteurs à fibre optique à ondes évanescentes utilisent essentiellement, pour des raisons de sensibilité, une fibre optique d'un diamètre relativement petit. La partie courbe est sujette à se briser du fait du matériau utilisé et de son faible diamètre.

Ainsi, il est connu du document FR2978547 de la demanderesse d'ajouter des moyens de protection contre les agressions mécaniques extérieures de la partie courbe de la fibre.

Ainsi, comme on le constate, l'amélioration de la sensibilité et la fiabilité du capteur sont des points cruciaux dans le développement des capteurs à fibres.

Enfin, dans le cas d'un guide d'onde de forme différente de celle d'une fibre optique, il peut être difficile voire impossible de modifier la courbure du guide d'onde.

Le document « Fiber evanescent wave spectroscopy using the mid-infrared provides useful fingerprints for metabolic profiling in humans » décrit l'utilisation de la technique de spectroscopie par fibre à ondes évanescentes pour l'exploration dans le moyen infra-rouge de la fonction de groupes chimiques dans un échantillon biologique.

Le document « From Selenium- to Tellurium-Based Glass Optical Fibers for Infrared Spectroscopies » décrit une classe particulière de fibres optiques pour réaliser des mesures par ondes évanescentes en spectroscopie infrarouge.

Le document US2011301047 décrit un dispositif de détection pour un fluide.

L'invention a notamment pour but d'apporter d'améliorer la sensibilité de détection de manière simple, efficace et économique.

A cette fin, elle propose un dispositif comprenant un support comportant un guide d'onde permettant la propagation de lumière à au moins une longueur d'onde générant vers l'extérieur des ondes évanescentes pour détecter des signatures optiques d'un milieu extérieur en contact avec la fibre optique, caractérisé en ce qu'il comprend des moyens de réception d'un échantillon liquide configurés pour réceptionner l'échantillon liquide au contact du guide d'onde de manière à imprégner le guide d'onde avec une partie de l'échantillon et des moyens actionnables de retrait du contact de l'échantillon liquide d'avec le guide d'onde.

Selon l'invention, le dispositif permet de recevoir l'échantillon liquide sur la fibre optique et d'y déposer les composés ou molécules cibles présents dans l'échantillon et destinés à être analysés. L'intégration de moyens de retrait actionnables permet d'enlever ultérieurement la goutte d'échantillon liquide. De cette manière, le solvant présent dans l'échantillon n'impacte plus la mesure comme c'est le cas dans l'art antérieur.

Si l'utilisation d'une fibre optique à partie courbe comme dans l'art antérieur n'est pas nécessaire, il est bien évidemment possible d'en utiliser une.

Selon une autre caractéristique de l'invention, lesdits moyens actionnables comprennent un organe mobile par rapport au support et qui comporte au moins une partie desdits moyens de réception, l'organe mobile étant déplaçable entre une première position dans laquelle lesdits moyens de réception sont aptes à recevoir l'échantillon liquide au contact du guide d'onde et une seconde position dans laquelle lesdits moyens de réception sont configurés pour que l'échantillon liquide ne soit plus en contact avec le guide d'onde.

Préférentiellement, l'organe mobile est monté déplaçable en translation sur le support entre sa première et sa seconde position et le support comprend des moyens de guidage à déplacement de l'organe mobile entre sa première position et sa seconde position.

Ces moyens de guidage peuvent être des plots en saillie sur le support et entre lesquels l'organe mobile peut se déplacer à translation.

Préférentiellement, le dispositif comprend des moyens de blocage du déplacement de l'organe mobile de la seconde position vers la première position lorsque l'organe mobile a été déplacé dans sa seconde position.

Il est souhaitable d'avoir de tels moyens pour éviter que l'organe mobile ne puisse revenir dans sa première position, ce qui pourrait conduire à ce que l'échantillon vienne de nouveau en contact avec la fibre optique. Cela permet également de s'assurer que chaque dispositif ne sera utilisé que pour le dépôt d'un seul échantillon et sera ensuite jeté après analyse, évitant ainsi les mélanges d'échantillons biologiques et les erreurs d'interprétation.

En pratique, l'opérateur met une goutte de liquide dans les moyens de réception prévus à cet effet, déplace l'organe mobile de sa première position vers sa seconde position dans laquelle l'organe mobile est empêché de retourner dans sa première position.

Dans une réalisation pratique de l'invention, lesdits moyens de blocage comprennent des ergots ou doigts anti-retour formés sur l'un du support ou de l'organe mobile et configurés pour servir de butée de déplacement sur l'autre du support ou de l'organe mobile lors du déplacement de l'organe mobile de sa seconde position vers sa première position.

Selon une autre caractéristique de l'invention, le dispositif comprend une partie formant couvercle sur le support et délimitant avec le support une enceinte logeant un tronçon de guide d'onde.

Selon encore une autre caractéristique de l'invention, le guide d'onde peut être fixé au support et le couvercle est scellé, par exemple par soudure, sur le support.

Dans une réalisation pratique de l'invention, l'organe mobile est monté déplaçable en translation suivant une direction sensiblement perpendiculaire à l'axe de propagation de la lumière dans le tronçon de guide d'onde, l'organe mobile comprenant une partie logée à l'intérieur de l'enceinte et qui comprend une cuvette de réception d'un échantillon liquide déplaçable entre le support et le tronçon de guide d'onde de sorte que dans la première position de l'organe mobile la cuvette soit agencée au droit du tronçon de guide d'onde et dans la seconde position la cuvette soit située à distance du tronçon de guide d'onde.

Avantageusement, le couvercle comprend une ouverture débouchant à l'intérieur de l'enceinte en regard de la cuvette de réception de l'organe mobile lorsque celui-ci est dans sa première position.

De cette manière, on comprend que l'opérateur injecte le liquide au moyen d'une pipette, par exemple, dans l'ouverture du couvercle afin que le liquide vienne se loger dans la cuvette de réception de l'organe mobile lorsque celui-ci est dans sa première position.

Préférentiellement, le guide d'onde est une fibre optique réalisée dans un matériau permettant la propagation d'une lumière à au moins une longueur d'onde infrarouge, tel qu'une fibre de verre de chalcogénure.

L'invention concerne encore un système de spectrométrie comportant un dispositif du type décrit ci-dessus.

L'invention concerne également un procédé de mise en oeuvre du dispositif décrit précédemment et qui consiste à :
a) disposer un échantillon liquide dans les moyens de réception ;
b) laisser la surface externe du guide d'onde s'imprégner de l'échantillon liquide durant un temps prédéterminé ;
c) actionner les moyens de retrait de manière à retirer le contact de l'échantillon liquide d'avec la fibre optique.

Dans une étape ultérieure du procédé, il consiste à disposer le dispositif obtenu à l'étape c) dans un système de spectrométrie pour analyse spectroscopique.

L'invention sera mieux comprise et d'autres détails, avantages et caractéristiques de l'invention apparaîtront à la lecture de la description suivante faite à titre d'exemple non limitatif, en référence aux dessins annexés dans lesquels :
- les figures 1 et 2 sont des vues schématiques du dessus des différentes pièces formant le dispositif selon l'invention ;
- la figure 3 est vue schématique à plus grande échelle de la zone entourée sur la figure 2 ;
- la figure 4A est une vue schématique en perspective du dispositif selon l'invention sans le couvercle ;
- la figure 4B est une vue schématique en perspective du couvercle du dispositif selon l'invention ;
- la figure 5 est une vue schématique en perspective du dispositif selon l'invention avec le couvercle ;
- la figure 6 est une vue schématique du dessus du dispositif selon l'invention sans le couvercle, l'organe mobile étant dans sa première position ;
- la figure 7 est une vue schématique du dessus du dispositif selon l'invention sans le couvercle, l'organe mobile étant dans sa seconde position ;
- la figure 8 est une vue schématique du dessus du dispositif selon l'invention avec le couvercle, l'organe mobile étant dans sa première position ;
- la figure 9 est une vue schématique du dessus du dispositif selon l'invention avec le couvercle, l'organe mobile étant dans sa seconde position ;
- les figures 10 et 11 illustrent le principe de fonctionnement/d'utilisation du dispositif selon l'invention ;
- les figures 12 et 13 sont des vues schématiques en perspective d'un système de spectroscopie comprenant le dispositif selon l'invention.

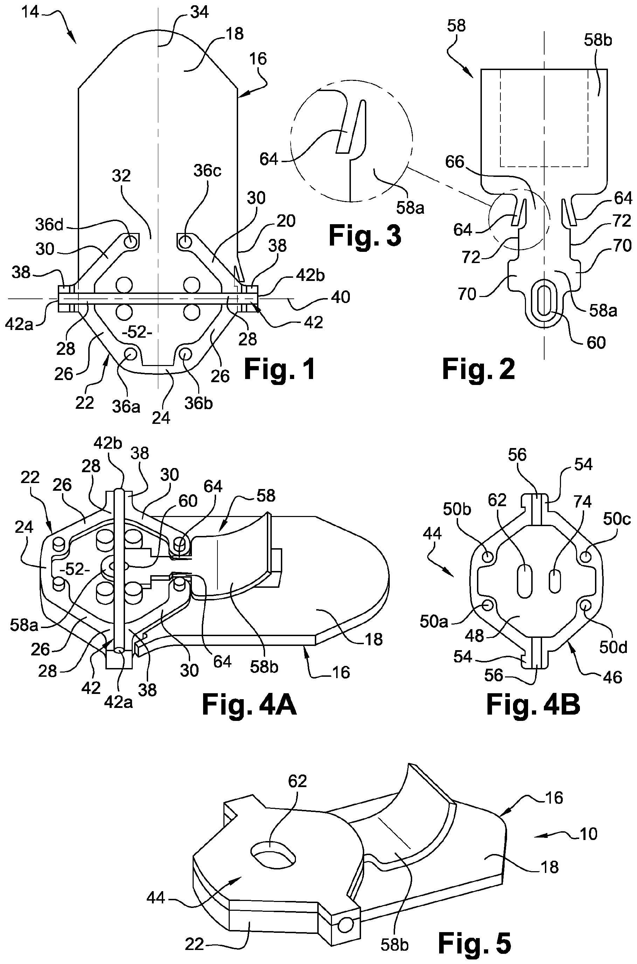

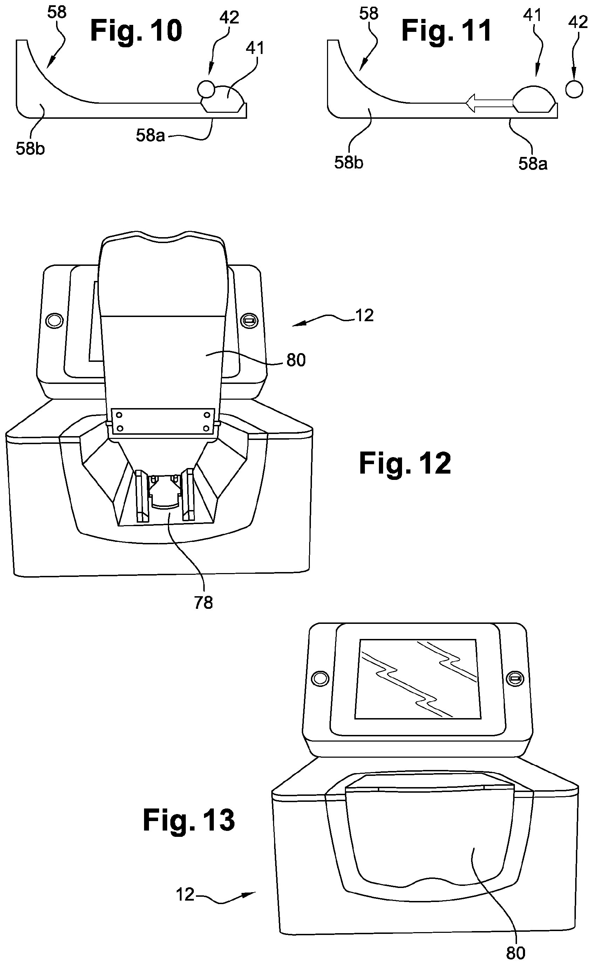

Les figures 1 à 9 illustrent une forme de réalisation d'un dispositif 10 à fibre optique selon l'invention et destiné à être utilisé avec un système de spectroscopie 12 comme représenté aux figures 12 et 13. Le dispositif comprend un support 14 comprenant une paroi support 16 de forme allongée comprenant une partie 18 dite de préhension et une partie opposée 20 destinée à former le capteur à fibre optique proprement dit. La partie de préhension 18 peut comprendre des stries parallèles (non représentées) facilitant la tenue en main du dispositif 10.

La partie à fibre optique du support comprend une nervure 22 définissant une forme polygonale et s'étendant sensiblement perpendiculairement à la paroi support 16. Cette nervure 22 comprend une première paroi 24 formée à l'extrémité du support allongé opposée à la partie de préhension 18 qui se prolonge à ses extrémités en deux secondes parois 26 s'éloignant l'une de l'autre lesquelles se prolongent en deux troisièmes parois 28 parallèles l'une à l'autre qui se prolongent par deux quatrièmes parois 30 convergentes l'une vers l'autre et définissant à leurs extrémités libres un espace 32 agencé en vis-à-vis de la première paroi 24 selon la direction allongée 34.

Les extrémités de la première paroi 24 et les extrémités libres des quatrièmes parois 30 portent des plots 36a, 36b, 36c, 36d en saillie dans un sens opposé à la paroi support.

Les troisièmes parois 28 portent chacune une patte latérale 38, les pattes latérales 38 s'étendant dans une même direction perpendiculaire 40 à la direction 34 mais dans des sens opposés et en s'éloignant l'une de l'autre.

Un tronçon de fibre optique 42 repose à ses extrémités 42a, 42b sur les pattes latérales 38 de manière à s'étendre selon la direction 40..

Comme représenté en figure 4B, le dispositif 10 comprend un couvercle 44 dont le pourtour porte un rebord 46 s'étendant perpendiculairement à une paroi de fond 48. Le rebord 46 forme un contour fermé destiné à venir en appui sur la nervure 22 du support 14. Ce rebord 46 comprend ainsi quatre trous borgnes 50a, 50b, 50c, 50d formés sur celui-ci de manière à venir coopérer avec les plots 36a, 36b, 36c, 36d des première 24 et quatrièmes 30 parois de la nervure 22 du support 14. Le couvercle 44 ainsi posé sur la nervure 22 du support 14 délimite avec la nervure 22 et la paroi support 16 une enceinte 52. Le couvercle 44 comprend également deux pattes latérales 54 aptes à venir se positionner sur les pattes 38 du support 14. Ces pattes latérales 54 comprennent chacune une encoche 56 à paroi courbe et dont la section est adaptée pour y recevoir intégralement la section du tronçon de fibre optique 42.

Selon l'invention, le dispositif comprend des moyens de réception d'une goutte au contact de la fibre optique et des moyens actionnables pour enlever cette goutte après contact avec le tronçon de fibre 42.

Dans la réalisation représentée aux dessins, les moyens actionnables sont formés par un organe mobile 58 comprenant une première partie 58a agencée à l'intérieur de l'enceinte et une seconde partie 58b agencée à l'extérieur de l'enceinte 52 et préhensible. L'organe mobile 58 a une forme allongée selon la direction 34 perpendiculaire à l'axe 40 du tronçon de fibre optique 42. La première partie 58a de l'organe mobile 58 comprend une cuvette 60 à son extrémité opposée à la seconde partie 58b. Cette cuvette 60 est destinée à recevoir l'échantillon liquide comme cela ressortira de la description ci-après. La hauteur de la nervure 22 du support ainsi que l'épaisseur de la première partie 58a de l'organe mobile 58 sont déterminés de manière à ce que l'espace entre la partie du tronçon de fibre optique à l'intérieur de l'enceinte 52 et la paroi support soit suffisant pour y introduire la première partie 58a de l'organe mobile 58.

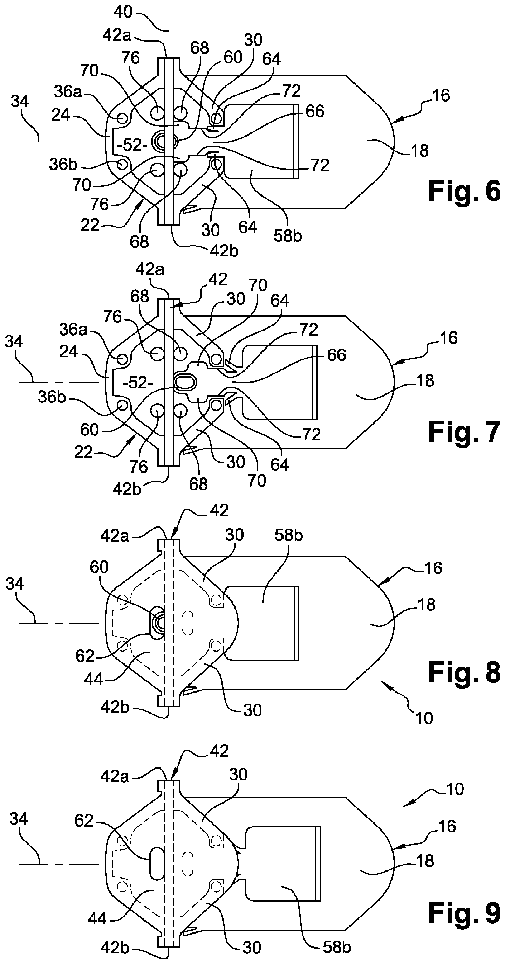

Comme représenté aux figures 6 et 7, l'organe mobile 58 est monté à translation sur le support 14 entre une première position (figure 6) dans laquelle la cuvette 60 est intercalée entre la fibre optique 42 et la paroi support 18 et une seconde position (figure 7) dans laquelle la cuvette 60 est éloignée du tronçon de fibre optique 42.

Afin de pouvoir insérer la goutte d'échantillon liquide dans la cuvette 60, le couvercle 44 comprend une ouverture qui 62 est agencée de manière à ce que lorsque l'organe mobile 58 est dans sa première position, la cuvette 60 est alors alignée avec l'ouverture (figure 8).

On remarque également (figure 8) que la fibre optique 42 n'est pas située en regard de l'ouverture 62 du couvercle 44 afin d'éviter tout endommagement par l'extrémité d'une pipette lors du dépôt de la goutte de liquide.

L'organe mobile 58 comprend des moyens anti-retours configurés pour empêcher l'organe mobile 58 de revenir dans sa première position lorsqu'il a été déplacé de première position initiale vers sa seconde position. Pour cela, il comprend des ergots 64 ou doigts anti-retour s'étendant de part et d'autre d'une partie de jonction 66 joignant la première partie 58a de l'organe mobile 58 et la seconde partie 58b de l'organe (figures 2, 4A, 6 et 7). Ces ergots 64 sont inclinés par rapport à l'axe 40 de manière à diverger l'un par rapport à l'autre dans le sens inverse du déplacement de l'organe mobile 58.

En pratique, lorsque l'organe mobile 58 est amené depuis sa première position vers sa seconde position, les ergots 64 initialement précontraints le long de la partie médiane 66 par les extrémités libres des quatrièmes parois 30, se déploient latéralement dès que la partie médiane 66 est sortie de l'enceinte 52, empêchant un retour de l'organe mobile 58 dans sa première position puisque les extrémités libres des ergots 64 viennent en appui sur les extrémités libres des quatrièmes parois 30.

Afin de faciliter le déplacement en translation de l'organe mobile 58, des moyens de guidage à déplacement sont prévus. Pour cela, l'intérieur de l'enceinte 52 comprend deux plots 68 entre lesquels des excroissances 70 latérale de la première partie 58a de l'organe mobile 58 sont aptes à venir en contact. De même, ces excroissances définissent deux épaulements venant en appuis sur les extrémités libres des quatrièmes parois 30 lorsque l'organe mobile 58 est dans sa seconde position, ce qui évite un désengagement de l'organe mobile 58 du support. L'organe mobile 58 est ainsi définitivement bloqué dans sa seconde position.

Les moyens de guidage comprennent également deux surfaces latérales 72 de la première partie 58a qui sont sensiblement parallèles et qui viennent en appui à coulissement sur les extrémités libres des quatrièmes parois 30 lors du déplacement de l'organe mobile 58 de sa première position vers sa seconde position.

Les moyens de guidage comprennent encore un plot 74 formé sur la surface de fond du couvercle 44 et dont la dimension est déterminée de manière à venir en contact avec la première partie 58a et empêcher tout basculement de l'organe mobile 58 lors de son déplacement qui pourrait endommager la fibre optique 42.

Les plots 68 ainsi que les deux autres plots additionnels 76 ont des hauteurs identiques et déterminées de manière à ce qu'ils viennent en contact avec le fond du couvercle 44 lorsque celui-ci est monté sur la nervure 22 du support.

La mise en place du dispositif selon l'invention s'effectue de la manière suivante : tout d'abord l'organe mobile 58 est agencé à l'intérieur de l'enceinte 52, puis le tronçon de fibre optique 42 est monté sur les pattes 38 de la nervure 22 selon l'axe 40 et enfin le couvercle 44 est monté sur la nervure 22 du support 14 et soudée à celle-ci par exemple par fusion avec la pointe d'un fer à souder. Une goutte d'échantillon biologique à analyser est insérer au travers de l'ouverture 62 du couvercle 44 afin de venir se loger dans la cuvette 60 de l'organe mobile 58 lequel est ensuite déplacé de sa première position (figure 10) vers sa seconde position dans laquelle la goutte de liquide n'est plus en contact avec la fibre optique 42 (figure 11) et dans laquelle l'organe mobile 58 est bloqué à déplacement sur le support 14.

Enfin, dans une dernière étape, le dispositif à fibre optique est agencé dans un logement 78 d'un système de spectroscopie 12 comprenant des moyens de connexion d'une 42b des extrémités du tronçon de fibre optique 42 à des moyens d'émission d'une lumière infrarouge et de l'autre extrémité 42a à des moyens d'analyse et de traitement. Le couplage de la lumière émise par les moyens d'émission avec l'extrémité 42a de la fibre optique ainsi que le couplage de la lumière sortant de la fibre optique peuvent être réalisés au moyen de lentilles.

Le système comprend un capot 80 destiné à fermer et rendre opaque le logement du dispositif à fibre optique lors de l'émission de la lumière infrarouge.

Sans sortir du cadre de l'invention, l'organe mobile pourrait consister une paroi de fond d'une cuvette qui serait déplaçable à rotation autour d'un axe entre une position fermée dans laquelle elle retient le liquide biologique et une position ouverte dans laquelle elle autorise l'évacuation du liquide biologique.

En pratique, les moyens de réception de l'échantillon biologique sont configurés pour recevoir un échantillon d'un volume compris entre 5 et 20 µL.

D'autres types de moyens de guidage sont également possible tels que des rainures et nervures complémentaires formées sur l'organe mobile et sur la paroi support.

Le dispositif peut également comprendre des moyens de chauffage de la fibre optique. Ces moyens sont avantageusement actionnés après retrait de la goutte d'échantillon liquide de manière à enlever les résidus liquide au contact de la fibre optique. Notons qu'une augmentation de la température à 35°C permet de raccourcir le temps de mesure de 50% en favorisant l'évacuation du solvant, par exemple de l'eau.

Selon une variante du dispositif, les moyens de chauffage peuvent également constituer les moyens de retrait de la goutte d'eau de sorte, ne nécessitant pas alors de moyens mobiles comme décrit précédemment.

Selon encore une autre variante du dispositif, les moyens de retrait de la goutte d'eau peuvent comprendre des moyens de soufflage comprenant par exemple de moyens d'amenée d'air au niveau de la zone de contact entre la fibre optique et la goutte d'eau. Le dispositif comprend alors également aussi des moyens d'évacuation de la goutte d'eau.

L'invention a été décrite en référence aux dessins en combinaison avec une fibre optique. On comprend toutefois qu'il serait possible d'utiliser tout type de guide d'onde permettant la propagation d'ondes et la génération d'ondes évanescentes. Ces guides d'ondes pouvant avoir par exemple une forme de lamelle telle que ceux utilisés dans les dispositifs de type biopuce à ADN bien connu de l'homme du métier.

## Revendications

1. Dispositif (10) comprenant un support (14) comportant un guide d'onde (42) permettant la propagation de lumière à au moins une longueur d'onde générant vers l'extérieur des ondes évanescentes, comprenant des moyens de réception d'un échantillon liquide configurés pour réceptionner l'échantillon liquide au contact du guide d'onde (42) de manière à imprégner le guide d'onde avec une partie de l'échantillon liquide et des moyens actionnables de retrait du contact de l'échantillon liquide d'avec du guide d'onde (42), **caractérisé en ce que** les moyens actionnables comprennent un organe mobile (58) par rapport au support (14) et qui comporte au moins une partie desdits moyens de réception (60), l'organe mobile (58) étant déplaçable entre une première position dans laquelle lesdits moyens de réception sont aptes à recevoir l'échantillon liquide au contact du guide d'onde et une seconde position dans laquelle lesdits moyens de réception (60) sont configurés pour que l'échantillon liquide ne soit plus en contact avec le guide d'onde (42).

2. Dispositif selon la revendication 1, **caractérisé en ce que** l'organe mobile (58) est monté déplaçable en translation sur le support (14) entre sa première et sa seconde position et **en ce que** le support (14) comprend des moyens de guidage (70, 72) à déplacement de l'organe mobile (58) entre sa première position et sa seconde position.

3. Dispositif selon la revendication 1 ou 2, **caractérisé en ce qu'**il comprend des moyens de blocage (64) du déplacement de l'organe mobile (58) de la seconde position vers la première position lorsque l'organe mobile (58) a été déplacé dans sa seconde position.

4. Dispositif selon la revendication 3, **caractérisé en ce que** lesdits moyens de blocage (64) comprennent des ergots ou doigts anti-retour formés sur l'un du support (14) ou de l'organe mobile (58) et configurés pour servir de butée de déplacement sur l'autre du support (14) ou de l'organe mobile (58) lors du déplacement de l'organe mobile (58) de sa seconde position vers sa première position.

5. Dispositif selon l'une des revendications précédentes, **caractérisé en ce qu'**il comprend une partie formant couvercle (44) sur le support et délimitant avec le support (14) une enceinte (52) logeant un tronçon de guide d'onde (42).

6. Dispositif selon l'une des revendications précédentes, **caractérisée en ce que** le guide d'onde (42) est fixé au support et le couvercle (44) est scellé, par exemple par soudure, sur le support.

7. Dispositif selon la revendication 5 ou 6, **caractérisé en ce que** l'organe mobile (58) est monté déplaçable en translation suivant une direction (34) sensiblement perpendiculaire à l'axe (40) de propagation de la lumière dans le tronçon de guide d'onde (42), l'organe mobile (58) comprenant une partie (58a) logée à l'intérieur de l'enceinte et qui comprend une cuvette (60) de réception d'un échantillon liquide déplaçable entre le support (14) et le tronçon de guide d'onde (42) de sorte que dans la première position de l'organe mobile la cuvette (60) soit agencée au droit du tronçon de guide d'onde (42) et dans la seconde position la cuvette (60) soit située à distance du tronçon de guide d'onde (42).

8. Dispositif selon la revendication 7, **caractérisé en ce que** le couvercle (44) comprend une ouverture (62) débouchant à l'intérieur de l'enceinte (52) en regard de la cuvette (60) de réception de l'organe mobile (58) lorsque celui-ci est dans sa première position.

9. Dispositif selon l'une des revendications précédentes, **caractérisé en ce qu'**il comprend des moyens de chauffage du guide d'onde.

10. Dispositif selon l'une des revendications précédentes, **caractérisé en ce que** le guide d'onde réalisé dans un matériau permettant la propagation d'une lumière à au moins une longueur d'onde infrarouge, tel qu'une fibre de verre de chalcogénure.

11. Système de spectrométrie pour analyse spectroscopique (12), **caractérisé en ce qu'**il comporte un dispositif selon l'une des revendications précédentes.

12. Procédé de mise en oeuvre du dispositif selon l'une des revendications 1 à 10, **caractérisé en ce qu'**il consiste à :
a) disposer un échantillon liquide dans les moyens de réception (60) ;
b) laisser la surface externe du guide d'onde s'imprégner de l'échantillon liquide durant un temps prédéterminé ;
c) actionner les moyens de retrait de manière à retirer le contact de l'échantillon liquide d'avec la fibre optique.

13. Procédé selon la revendication 12, **caractérisé en ce qu'**il consiste à disposer le dispositif obtenu à l'étape c) dans un système de spectrométrie (12) selon la revendication 11.

## Patentansprüche

1. Vorrichtung (10), enthaltend einen Halter (14), der zumindest einen Wellenleiter (42) aufweist, der die Ausbreitung von Licht auf zumindest einer Wellenlänge gestattet, das evaneszente Wellen nach außen erzeugt, enthaltend
Mittel zum Aufnehmen einer Flüssigprobe, die dazu ausgelegt sind, die Flüssigprobe im Kontakt mit dem Wellleiter (42) aufzunehmen, so dass der Wellenleiter mit einem Teil der Flüssigprobe getränkt wird, sowie betätigbare Mittel zum Aufheben des Kontakts der Flüssigprobe mit dem Wellenleiter (42), **dadurch gekennzeichnet, dass** die betätigbaren Mittel ein bezüglich des Halters (14) bewegliches Glied (58) enthalten, das zumindest einen Teil der Aufnahmemittel (60) enthält, wobei das bewegliche Glied (58) zwischen einer ersten Position, in welcher die Aufnahmemittel die Flüssigprobe im Kontakt mit dem Wellenleiter aufnehmen können, und einer zweiten Position verlagerbar ist, in welcher die Aufnahmemittel (60) so ausgelegt sind, dass die Flüssigprobe nicht mehr mit dem Wellenleiter (42) in Kontakt ist.

2. Vorrichtung nach Anspruch 1, **dadurch gekennzeichnet, dass** das bewegliche Glied (58) an dem Halter (14) zwischen seiner ersten und seiner zweiten Position translatorisch verschiebbar gelagert ist, und dass der Halter (14) Führungsmittel (70, 72) zum Verlagern des beweglichen Glieds (58) zwischen seiner ersten Position und seiner zweiten Position aufweist.

3. Vorrichtung nach Anspruch 1 oder 2, **dadurch gekennzeichnet, dass** sie Blockiermittel (64) zum Blockieren der Verlagerung des beweglichen Glieds (58) von der zweiten Position in die ersten Position, wenn das bewegliche Glied (58) in seine zweite Position verlagert wurde, aufweist.

4. Vorrichtung nach Anspruch 3, **dadurch gekennzeichnet, dass** die Blockiermittel (64) Vorsprünge bzw. Zapfen als Rücklaufsperre enthalten, die an einem aus Halter (14) bzw. beweglichem Glied (58) ausgebildet und dazu ausgelegt sind, als Verlagerungsanschlag am anderen aus Halter (14) bzw. beweglichem Glied (58) bei der Verlagerung des beweglichen Glieds (58) von seiner zweiten Position in seine erste Position zu dienen.

5. Vorrichtung nach einem der vorangehenden Ansprüche, **dadurch gekennzeichnet, dass** sie einen Abdeckungsteil (44) an dem Halter aufweist, der mit dem Halter (14) einen Raum (52) eingrenzt, welcher einen Wellenleiterabschnitt (42) aufnimmt.

6. Vorrichtung nach einem der vorangehenden Ansprüche, **dadurch gekennzeichnet, dass** der Wellenleiter (42) am Halter befestigt und die Abdeckung (44) beispielsweise durch Verschweißen am Halter angebracht ist.

7. Vorrichtung nach Anspruch 5 oder 6, **dadurch gekennzeichnet, dass** das bewegliche Glied (58) in einer Richtung (34) im Wesentlichen senkrecht zur Ausbreitungsachse (40) des Lichts in dem Wellenleiterabschnitt (42) translatorisch verschiebbar gelagert ist, wobei das bewegliche Glied (58) ein Teil (58a) aufweist, das innerhalb des Raums aufgenommen ist und eine Mulde (60) zum Aufnehmen einer Flüssigprobe aufweist, die zwischen dem Halter (14) und dem Wellenleiterabschnitt (42) verlagerbar ist, so dass in der ersten Position des beweglichen Glieds die Mulde (60) im Bereich des Wellenleiterabschnitts (42) angeordnet ist und in der zweiten Position die Mulde (60) im Abstand vom Wellenleiterabschnitt (42) liegt.

8. Vorrichtung nach Anspruch 7, **dadurch gekennzeichnet, dass** die Abdeckung (44) eine Öffnung (62) aufweist, die innerhalb des Raums (52) entgegengesetzt zur Mulde (60) zum Aufnehmen des beweglichen Glieds (58) ausmündet, wenn dieses in seiner ersten Position ist.

9. Vorrichtung nach einem der vorangehenden Ansprüche, **dadurch gekennzeichnet, dass** sie Mittel zum Erhitzen des Wellenleiters aufweist.

10. Vorrichtung nach einem der vorangehenden Ansprüche, **dadurch gekennzeichnet, dass** der Wellenleiter aus einem Material hergestellt ist, das die Ausbreitung von Licht auf zumindest einer Infrarotwellenlänge gestattet, wie etwa Chalcogenid-Glasfaser.

11. Spektrometriesystem zur spektroskopischen Analyse (12), **dadurch gekennzeichnet, dass** es eine Vorrichtung nach einem der vorangehenden Ansprüche aufweist.

12. Verfahren zum Herstellen der Vorrichtung nach einem der Ansprüche 1 bis 10, **dadurch gekennzeichnet, dass** es darin besteht,
a) eine Flüssigprobe in die Aufnahmemittel (60) einzubringen;
b) die Außenfläche des Wellenleiters mit der Flüssigprobe für eine vorbestimmte Zeit zu tränken;
c) die Aufhebungsmittel so zu betätigen, dass der Kontakt der Flüssigprobe mit der Lichtleitfaser aufgehoben wird.

13. Verfahren nach Anspruch 12, **dadurch gekennzeichnet, dass** es darin besteht, die in Schritt c) erhaltene Vorrichtung in ein Spektrometriessystem (12) nach Anspruch 11 anzuordnen.

## Claims

1. A device (10) comprising a support (14) having a wave guide (42) enabling the propagation of light in at least one wavelength, generating evanescent waves outwards, comprising means for receiving a liquid sample, so configured as to receive the liquid sample upon contact with the wave guide (42) so as to impregnate the wave guide with a portion of the liquid sample, and actuatable means for breaking the contact between the liquid sample and the wave guide (42) **characterized in that** the actuatable means comprise a member (58) which is movable relative to the support (14) and which comprises at least a portion of said receiving means (60), with the movable member (58) being movable between a first position in which said receiving means are adapted to receive the liquid sample upon contact with the wave guide and a second position in which said receiving means (60) are so configured that the liquid sample is no longer in contact with the wave guide (42).

2. A device according to claim 1, **characterized in that** the movable member (58) is so mounted as to be movable in translation on the support (14) between the first and the second position thereof and **in that** the support (14) comprises means for guiding (70, 72) the motion of the movable member (58) between the first and the second position thereof.

3. A device according to claim 1 or 2, **characterized in that** it comprises means (64) for blocking the motion of the movable member (58) from the second position to the first position when the movable member (58) has been moved into the second position thereof.

4. A device according to claim 3, **characterized in that** said blocking means (64) comprise non-return pins or fingers formed on one of the support (14) or the movable member (58), and so configured as to be used as displacement stops on the other one of the support (14) or the movable member (58) upon the movable member (58) moving from the second position to the first position thereof.

5. A device according to one of the preceding claims, **characterized in that** it comprises a part forming a cover (44) on the support and defining an enclosure (52) accommodating a wave guide section (42) with the support (14).

6. A device according to one of the preceding claims, **characterized in that** the wave guide (42) is attached to the support and the cover (44) is sealed on the support, for instance by welding.

7. A device according to claim 5 or 6, **characterized in that** the movable member (58) is so mounted as to be movable in translation along a direction (34) substantially perpendicular to the axis (40) of propagation of light in the wave guide (42) section, with the movable member (58) comprising a portion (58a) accommodated inside the enclosure and which comprises a cup (60) for receiving a liquid sample which is movable between the support (14) and the wave guide section (42) so that, in the first position of the movable member, the cup (60) is arranged at right angles with the wave guide section (42) and in the second position, the cup (60) is located at a distance from the wave guide section (42).

8. A device according to claim 7, **characterized in that** the cover (44) comprises an aperture (62) opening inside the enclosure (52) opposite the receiving cup (60) of the movable member (58) when the latter is in the first position thereof.

9. A device according to one of the preceding claims, **characterized in that** it comprises means for heating the wave guide.

10. A device according to one of the preceding claims, **characterized in that** the wave guide is made of a material enabling the propagation of light in at least one infrared wavelength, such as chalcogenide glass fiber.

11. A spectrometric system for spectroscopic analysis (12), **characterized in that** it comprises a device according to one of the preceding claims.

12. A method for implementing the device according to one of claims 1 to 10, **characterized in that** it consists in:
a) positioning a liquid sample in the receiving means (60);
b) letting the outer surface of the wave guide impregnate with the liquid sample for a predetermined time;
c) actuating the contact breaking means so as to break the contact between the liquid sample and the optical fiber.

13. A method according to claim 12, **characterized in that** it consists in positioning the device obtained in step c) in a spectrometric system (12) according to claim 11.
